# EUROPEAN PATENT SPECIFICATION

(11) **EP 3 970 692 B1**
(45) Date of publication and mention of the grant of the patent: **12.02.2025**
(21) Application number: 20196886.4
(22) Date of filing: 18.09.2020
(51) Int. Cl.: A61K 9/00, A61K 31/775, A61K 47/10, A61K 47/26, A61K 47/32

(54) **LIPID-BASED OPHTHALMIC COMPOSITION FOR THE TREATMENT OF DRY EYE**
LIPIDBASIERTE OPHTHALMISCHE ZUSAMMENSETZUNG ZUR BEHANDLUNG VON TROCKENEM AUGE
COMPOSITION OPHTALMIQUE À BASE DE LIPIDES POUR LE TRAITEMENT DE LA SÉCHERESSE OCULAIRE

(43) Date of publication of application: 23.03.2022
(73) Proprietor: Omnivision GmbH, 82178 Puchheim (DE)
(72) Inventor: KOHL, Tobias, 82178 Puchheim (DE); HOFFMANN, Patrick, 82178 Puchheim (DE); HOFFMANN, Burkhardt, 82178 Puchheim (DE); MÜLLER, Saskia, 82178 Puchheim (DE); GASCO, Paolo, 10144 Torino (IT)
(74) Representative: Wächtershäuser & Hartz Patentanwaltspartnerschaft mbB

(56) References cited:
- EP-A1- 2 404 593
- WO-A2-2006/050838
- TAMILVANAN S ET AL: "The potential of lipid emulsion for ocular delivery of lipophilic drugs", EUROPEAN JOURNAL OF PHARMACEUTICS AND BIOPHARMACEUTICS, ELSEVIER SCIENCE PUBLISHERS B.V., AMSTERDAM, NL, vol. 58, no. 2, 1 September 2004 (2004-09-01), pages 357 - 368, XP004526318, ISSN: 0939-6411, DOI: 10.1016/J.EJPB.2004.03.033
- LALLEMAND FREDERIC ET AL: "Successfully Improving Ocular Drug Delivery Using the Cationic Nanoemulsion, Novasorb", JOURNAL OF DRUG DELIVERY, vol. 2012, 27 February 2012 (2012-02-27), pages 1 - 16, XP055772933, ISSN: 2090-3014, Retrieved from the Internet <URL:http://downloads.hindawi.com/archive/2012/604204.xml> DOI: 10.1155/2012/604204

## Description

### FIELD OF THE INVENTION

The invention relates to an ophthalmic composition, notably an ophthalmic oil-in-water emulsion composition. The emulsion comprises at least one ophthalmically acceptable oil comprising castor oil, tyloxapol, poloxamer 188, and polyoxyethylene (20) sorbitan mono-oleate. The invention also provides a process for preparing the composition.

### BACKGROUND OF THE INVENTION

Several components of the ocular surface contribute to maintaining and protecting a smooth refractive layer to facilitate the optimal eyesight. At the air-water interface, the tear film lipid layer (TFLL), a mixture of lipids and proteins, plays a key role in tear surface tension and is important for the physiological hydration of the ocular surface and for ocular homeostasis. Alterations in tear fluid rheology, differences in lipid composition, or downregulation of specific tear proteins are found in most types of ocular surface disease, including dry eye disease (DED). Artificial tears have long been a first line of treatment in DED and aim to replace or supplement tears. More recently, lipid-containing eye drops have been developed to more closely mimic the combination of aqueous and lipid layers of the TFLL. Several lipid-based products are known in the treatment of DED, such as liposome lid sprays, emulsion eye drops, and other lipid-containing compositions.

WO 2006/050838 A2 discloses ophthalmic cationic oil-in-water type emulsions having a zeta potential remaining positive over time. Lallemand et al., Journal of Drug Delivery, 2012, Article ID 604204, discloses topical ophthalmic delivery of active ingredients using cationic nano emulsions. Both documents consider high pressure homogenization (HPH) as a necessary process step to obtain suitable nano emulsions. HPH is typically performed by forcing a liquid through a narrow nozzle at high pressure and by such establishing high shear stress. Typical pressures are between 1500 and 4000 bars. This can be used to stabilize bio-oil as emulsions, and the droplet size can be adjusted by the levels of pressure and energy input. However, HPH is device-intensive and expensive.
EP 2404593 A1 discloses ophthalmic oil-in-water emulsions containing compositions containing quaternary ammonium compounds in which the nitrogen atom is substituted by at least one alkyl group having at least 12 carbon atoms.
S. Tamilvanan et al., European Journal of pharmaceutics and biopharmaceutics, (20040901), vol. 58, no. 2, pages 357 - 368 discloses lipid emulsions for ocular delivery of lipophilic drugs.

Nano emulsions are currently considered the best solution to improving the ocular delivery of ophthalmic drugs. Some reasons for only few marketed products based on nano emulsions are technical issues such as stability of colloidal systems, requirement for new excipients or use of organic solvents noncompliant to regulatory standards, unknown or unacceptable toxicity profiles, or unique scale-up and manufacturing requirements.

In the preparation of ophthalmic oil-in-water emulsions, the major technical challenge is the reliable preparation of the emulsion, in particular a nano emulsion, which should be as little device-intensive as possible, as well as its stability.

Departing from the prior art, it is an object of the invention to provide an ophthalmic oil-in-water emulsion that has, on the one hand, suitable properties as regards stability and effectivity, and on the other hand can be produced as little device-intensive as possible. It is another object of the invention to provide a suitable process for the preparation of such an ophthalmic oil-in-water emulsion.

### SUMMARY OF THE INVENTION

In order to accomplish these objects, the invention provides:
An ophthalmic oil-in-water emulsion comprising a) at least one ophthalmically acceptable oil, wherein the ophthalmically acceptable oil comprises castor oil, b) tyloxapol, c) poloxamer 188, and d) polyoxyethylene (20) sorbitan mono-oleate.

The invention further provides a process for the preparation of an ophthalmic oil-in-water emulsion comprising the steps of mixing at least components a) to d), namely a) at least one ophthalmically acceptable oil, wherein the ophthalmically acceptable oil comprises castor oil, b) tyloxapol, c) poloxamer 188, and d) polyoxyethylene (20) sorbitan mono-oleate; and emulsifying the mixture by high shear mixing to obtain an emulsion, preferably a nano emulsion.

The inventors have surprisingly found that a combination of at least three specific surfactants, namely tyloxapol, a poloxamer having an HLB value equal to or higher than 18, and a non-ionic surfactant having an HLB value in the range of from 12 to 17 is superior to known surfactant systems to obtain suitable properties as regards stability and effectivity of the obtainable emulsion, and on the other hand can be produced as little device-intensive as possible. In particular, it has been surprisingly found that when said at least three surfactants are used, the step of high pressure homogenization, which was considered in the art as necessary process step, can be avoided and that rather high shear mixing is sufficient to obtain suitable and stable nano emulsions.

### BRIEF DESCRIPTION OF THE FIGURES

Fig. 1 shows the oil droplet distribution (number of measurements n=3) of an emulsion of the invention.
Fig. 2 shows the oil droplet distribution (n=3) of comparative emulsion 1.

### DETAILED DESCRIPTION OF THE INVENTION

The ophthalmic oil-in-water emulsion of the invention comprises at least one ophthalmically acceptable oil and the surfactants.

The term "ophthalmic" refers to a composition suitable for use in ophthalmology, notably suitable for topical application to the surface of the eyes. The ophthalmic composition of the invention is generally an aqueous emulsion, namely an oil-in-water emulsion of the components mentioned in an aqueous solvent, preferably water. The water is present in an amount adding up to 100% (termed "ad 100%"). Examples of the ophthalmic compositions are eye drops or eye sprays. Preferred are eye drops.

The oil-in-water emulsion of the invention comprises at least one ophthalmically acceptable oil, namely castor oil. Suitable ophthalmically acceptable oils are known in the art. Typical ophthalmically acceptable oils are selected from known pharmaceutically acceptable oils, e.g. animal oils, vegetable oils, synthetic oils or mixtures thereof. Preferably, the oil comprises pharmaceutically acceptable fatty acid esters, e.g. fatty triglycerides or fatty acid monoesters. More preferably are long chain triglycerides, i.e. based on fatty acids having a C₁₃₋₂₁ alkyl chain. Even more preferably, the ophthalmically acceptable oil substantially consists, e.g. is only, castor oil, and no other oils, such as mineral oil or medium chain triglycerides, i.e. based on fatty acids having a C₆₋₁₂ alkyl chain, are present.

In one embodiment, the content of the ophthalmically acceptable oil in the oil-in-water emulsion is about 0.5 wt% to about 2 wt%, more preferably about 0.6 wt% to about 1.6 wt %, in particular about 0.7 wt% to about 1.2 wt%, e.g. about 0.9 wt%.

Preferably the emulsion has a substantially neutral to negative zeta potential (determined in 10 mM NaCl solution), i.e. a zeta potential between -30 mV and 0 mV, preferably between -20 mV and -2 mV and more preferably between -10 mV and -4 mV.

The oil-in-water emulsion comprises at least three different surfactants, namely tyloxapol, a poloxamer having an HLB value equal to or higher than 18, namely poloxamer 188, and a non-ionic surfactant having an HLB value in the range of from 12 to 17, namely polyoxyethylene (20) sorbitan mono-oleate.

Tyloxapol is a known nonionic liquid polymer of the alkyl aryl polyether alcohol type having the following chemical structure: 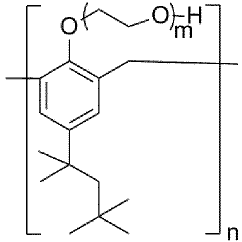 Index m is typically 6 to 8, and index n is typically equal to or smaller than 5.
Tyloxapol is mainly used in the art as a surfactant to aid liquefaction and removal of mucopurulent bronchopulmonary secretions, but is also known as surfactant in ophthalmic emulsions. Tyloxapol is commercially available.

The content of the tyloxapol in the oil-in-water emulsion is about 0.1 wt% to about 1 wt%, more preferably about 0.2 wt% to about 0.6 wt %, in particular about 0.25 wt% to about 0.5 wt%, e.g. about 0.3 wt%.

Poloxamers are nonionic triblock copolymers composed of a central hydrophobic chain of polyoxypropylene (i.e. polypropylene oxide)) flanked by two hydrophilic chains of polyoxyethylene (i.e. poly(ethylene oxide)). Poloxamers have the following chemical structure: 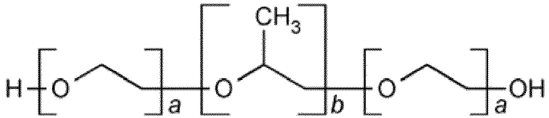 Typical values for the indexes are for a about 2 to about 130, and for b about 15 to about 67. Typical suitable and commercially used poloxamers having an HLB value equal to or higher than 18 are: poloxamer 188 (a=75, b=30) (HLB=29), poloxamer 237 (a=64, b=37) (HLB >24), poloxamer 338 (a=141, b=44) (HLB > 24), and poloxamer 407 (a=98-101, b=56) (HLB=22). The poloxamer used in the emulsion of the invention is poloxamer 188 (a=75, b=30) (HLB=29). Most preferably, only poloxamer 188 is used as poloxamer in the emulsion of the invention. Suitable poloxamers are commercially available, e.g. under the tradenames Pluronic, Pluracare and Synperonic.

The HLB values from suitable, in particular from commercially available surfactants, are known in the art. The HLB can be determined as described below.

The content of the poloxamer, preferably of poloxamer 188, in the oil-in-water emulsion is about 0.03 wt% to about 1 wt%, more preferably about 0.05 wt% to about 0.5 wt %, in particular about 0.08 wt% to about 0.2 wt%, e.g. about 0.1 wt%.

The third surfactant of item d) used in the emulsions of the invention is polyoxyethylene (20) sorbitan monooleate (Tween 80^{®}), most preferred as the only third surfactant having an HLB value in the range of from 12 to 17.

The content of polyoxyethylene (20) sorbitan monooleate in the oil-in-water emulsion is about 0.05 wt% to about 1 wt%, more preferably about 0.1 wt% to about 0.6 wt %, in particular about 0.15 wt% to about 0.3 wt%, e.g. about 0.2 wt%.

In a preferred embodiment, the oil-in-water emulsion of the invention comprises an ophthalmically acceptable cosurfactant. Suitable cosurfactants are medium (C6-C12) and long chain (C12-C18) alcohols, propylene glycol, polyethylene glycol, e.g. PEG 200, and glycerol, the latter being most preferred. The content of the cosurfactant, preferably of glycerol, in the oil-in-water emulsion is about 0.1 wt% to about 3 wt%, more preferably about 0.5 wt% to about 2.5 wt %, in particular about 1 wt% to about 2 wt%, e.g. at least 1.5 wt%.

The oil-in-water emulsion of the invention is preferably free from cationic surfactants, anionic surfactants, and more preferably free from cationic surfactants, anionic surfactants, short-chain, e.g. C₁₋₄ monohydric alcohols, fatty acids, e.g. C₄₋₈ fatty acids and/or from the group of lecithins or/and phospholipids.

The emulsion of the invention may comprise other agents commonly used in ophthalmic formulations, e.g. buffer agents such as citrate salts, phosphate salts, etc., osmolarity agents (also known in the art as isotonic agents), viscosity-increasing compounds, osmoprotectants, antimicrobial preservatives, antioxidants, or stabilizers. Preferably, the emulsion of the invention is free of preservatives.

The ophthalmic emulsion of the present invention may comprise an ophthalmically acceptable buffer, which is typically present in the aqueous phase. Examples of an ophthalmically acceptable buffer include citrate buffer, tris (trometamol) buffer, acetate buffer, borate buffer, and phosphate buffer. Preferred buffers are citrate buffer and tris buffer, most preferred is tris buffer. The content of the buffer in the ophthalmic composition of the invention may be from about 0.001 wt% to about 1 wt%, preferably from about 0.01 wt% to about 0.1 wt%, more preferably from about 0.02 wt% to about 0.05 wt%.

In a preferred embodiment, the ophthalmic composition of the present invention has a pH value between 6.8 to 8.0, preferably between 7.0 and 7.8, more preferably between 7.2 and 7.6. In an exemplary embodiment, the buffer is tris buffer with a pH at 7.4.

In one preferred embodiment, the oil-in-water emulsion comprises at least one osmolarity agent.

The term "osmolality" relates to the concentration of solutes in a solution and is defined as the number of osmoles (Osm) of solute per kilogram of solution in unit Osm/kg or mOsm/kg. The osmole (Osm) is a unit of measurement which defines the number of moles of solute(s) that contribute(s) to the osmotic pressure of a solution. The osmolality (and osmolarity) of small molecules can generally be calculated from their concentration in solution. In the case of simple salts, such as sodium chloride, which dissociate completely in water, both the (sodium) cation and the (chloride) anion contribute to the osmolality, and the osmolality can be calculated approximately as the sum of the concentrations of cations and anions. In the case of complex salts such as sodium phosphate, which partially dissociate in water, the osmolality depends on concentration and pH. Therefore, herein, osmolality is determined experimentally and numerical values of osmolality are measured values according to Ph. Eur. 2.2.35.

The similar term "osmolarity" also relates to a measure of the concentration of solute(s) in a solution and is defined as the number of osmoles (Osm) of solute per liter of solution in unit Osm/L or mOsm/L. Thus, osmolality and osmolarity of a given aqueous composition are linked by the density of the composition.

The term "osmolarity agent" generally refers to a substance that dissolves in the water of the composition of the invention and thus contributes to the osmolality (and osmolarity) of the composition. Herein, the at least one osmolarity agent contributes the major part of the osmolality to the composition, e.g. at least 90%, preferably at least 95%, and more preferably at least 97% of the osmolality. The term "osmolarity agent" comprises both penetrating and non-penetrating solutes, i.e. solutes than can and cannot, respectively, penetrate a membrane of an osmotic cell. Examples of osmolarity agents are inorganic soluble salts such as sodium and potassium salts (e.g. sodium chloride, potassium chloride), mono- and disaccharides (e.g. trehalose), and sugar alcohols. Examples of sugar alcohols are glycerol, mannitol, xylitol, and sorbitol. One osmolarity agent may be used alone in the ophthalmic composition of the invention, or two or more may be used in combination. For example, inorganic soluble salts and sugar alcohols may be used in combination as the at least one osmolarity agent. Preferably, the at least one osmolarity agent is or comprises one or more sugar alcohol(s). More preferably, the at least one osmolarity agent is or comprises glycerol. In case, sodium chloride is present, the content of sodium chloride is preferably below 0.1 wt %, more preferably below 0.05 wt %, in particular below 0.01 wt %.

The at least one osmolarity agent should be present in the ophthalmic composition of the invention so as to achieve the desired osmolality. The osmolality may be at least 50 mOsm/kg, preferably at least 100 mOsm/kg, more preferably at least 120 mOsm/kg, e.g. about 150 mOsm/kg. The upper limit of the osmolality is not specifically limited. However, the osmolality is generally not more than 320 mOsm/kg, preferably not more than 250 mOsm/kg to avoid excessive osmotic shock and/or burning sensation upon application to an eye.

As mentioned above, the at least one osmolarity agent is preferably a sugar alcohol, notably glycerol. The sugar alcohol, which is both osmolarity agent and cosurfactant, may be present in an amount of from 0.5 to 3.5 % by weight, preferably from 1.0 to 3.0 % by weight, and even more preferably from 1.5 to 2.0 % by weight of the ophthalmic composition. The content of osmolarity agent other than cosurfactant, in particular other than sugar alcohol (particularly glycerol), such as mono- and disaccharides (e.g. trehalose), may be from 0.1 to 2 % by weight, preferably from 0.3 to 1.5 % by weight, and even more preferably from 0.4 to 1.0 % by weight of the ophthalmic composition. A total content of glycerol exceeding 3.5 % by weight is not preferred, since such content can cause excessive dehydration of ocular tissue, and a content of the glycerol below 1.5 % by weight is not preferred since such content may result in insufficient osmolality and/or cosurfactant effect. Within the ranges mentioned, sufficiently high osmolality may be achieved while an excessive burning effect or eye irritation upon application of the ophthalmic composition to the eye can be avoided.

The emulsion of the present invention is preferably a micro emulsion, wherein the average size of the oil droplets is less than 1 µm, more preferably a nano emulsion. Nano emulsion as referred to herein means that the droplet size of the oil droplets is less than 500 nm, preferably about 100 nm to about 300 nm. Oil droplet size can be determined as known in the art, preferably by photo-optical methods, such as a dynamic light scattering technique, e.g. using a Malvern Zetasizer nano ZS, Submicron Particle Size Analyzer N5 Beckman Coulter, or similar devices.

The emulsion of the present invention preferably has a PDI (polydispersity index) equal to or below 0.4, more preferably equal to or below 0.35. The term "polydispersity" (or "dispersity" as recommended by IUPAC) is used to describe the degree of non-uniformity of a size distribution of particles. Also known as the heterogeneity index, PDI is dimensionless and is basically a representation of the distribution of size populations within a given sample. The numerical value of PDI ranges from 0.0 (for a perfectly uniform sample with respect to the particle size) to 1.0 (for a highly polydisperse sample with multiple particle size populations). Values of 0.2 and below are most commonly deemed acceptable in practice for polymer-based nanoparticle materials. In drug delivery applications using lipid-based carriers, such as liposome and nanoliposome formulations, a PDI of 0.3 and below is known to be considered as acceptable and indicates a homogenous population of particles, such as oil droplets or liposomes

It is further preferred that the emulsion of the present invention is stable upon storage for 6 months, i.e. does not change its physical state during 6 months storage, at a temperature range between 5° and 40°C, and particularly does not show a significant increase in oil droplet size nor phase separation.

Any amounts/contents indicated herein are % by weight (w/w), based on the total weight of the emulsion, if not indicated otherwise.

In one preferred embodiment, the oil-in-water emulsion comprises at least one osmoprotectant (also known in the art as compatible solutes), i.e. a small organic molecule with neutral charge and low toxicity at high concentrations that acts as osmolytes and helps organisms survive extreme osmotic stress. Suitable compounds are known in the art, e.g. betaines and associated molecules, sugars, polyols, and amino acids. Preferred examples are betaine (glycine betaine), glycine, ectoine, taurine, sorbitol, trehalose, sarcosine, raffinose, trimethylamine-N-oxide, glycerol, propylenglycole, butylenglycole, urea, dicycandiamide, L-carnithine, erythritol, myo-inositol and/or dimethyl sulfoniopropionate, more preferably glycine betaine, myo-inositol, taurine, ectoine and/or trimethylamine-N-oxide, as single compound or in combination thereof. The osmoprotectant may be present in an amount of from about 0.01 to 5 % by weight, preferably from about 0.1 to 2.0 % by weight, and even more preferably from about 0.2 to 1.0 % by weight of the ophthalmic composition.

In one embodiment, the oil-in-water emulsion comprises at least one viscosity regulating agent.

The viscosity regulating agent used in the emulsion of the invention may also be referred to as "thickener", "viscosity improver" or "gelling agent". The viscosity regulating agent is a polymeric hydrophilic compound that is soluble in water, thereby increasing the viscosity of the solution. It has film-building properties when applied to the eye, which protects the cornea and increases the remaining time of the ophthalmic composition on the cornea. The viscosity regulating agent may be a hydroxyalkylated cellulose, alkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, or salts of any of these. The hydroxyalkylated cellulose may be hydroxypropylcellulose or hydroxyethylcellulose. The alkylated cellulose may be methylcellulose or ethylcellulose. Further, a viscosity regulating agent may be hydroxypropylmethylcellulose (hypromellose, HPMC), however, in one embodiment, the ophthalmic composition of the invention does not contain HPMC. Two or more viscosity regulating agents may be combined in the ophthalmic composition of the invention. Among the above viscosity regulating agents, hyaluronic acid or a salt thereof and cross-linked hyaluronic acid or a salt thereof are preferred and hyaluronic acid or a salt thereof is most preferred.

The ophthalmic emulsion of the invention may comprise the one or more viscosity regulating agent(s), preferably said hyaluronic acid and/or a hyaluronate, in an amount of from about 0.001 % to about 0.1 % by weight, preferably from about 0.01 % to about 0.06% by weight, more preferably from about 0.015 % to 0.04 % by weight, and most preferably from 0.020 to 0.035% by weight of the ophthalmic emulsion. These concentration ranges relate to the sum of viscosity regulating agents, if two or more different ones are used. Within the above range, the ophthalmic composition such as eye drop formulation is comfortable to apply and suppresses eye irritation. The exact concentration may be chosen so as to reach a desired viscosity of the ophthalmic composition. The viscosity regulating agent may be used alone or in combination of two or more kinds. Among the above, hyaluronic acid and salts thereof is particularly preferred from the view of film stability on the eye and suppression of eye irritation.

The molecular weight of the viscosity regulating agent may be chosen so as to adjust the viscosity of the ophthalmic composition to a desired range. The molecular weight may be within the range of from 100,000 to 10,000,000 Dalton, preferable within the range of from 500,000 to 5 000,000 Dalton, more preferably within the range of from 800,000 to 4,000,000 Dalton, and most preferably within the range of from 1,000,000 to 3,000,000 Dalton.

These molecular weights may be combined with the content of the one or more viscosity regulating agent(s) described above. The content then refers to the amount of the viscosity regulating agent in the molecular weight range given. For example, the ophthalmic composition may contain from 0.01 to 0.05 % by weight of one or more viscosity regulating agent, preferably of hyaluronic acid and/or a hyaluronate, having a molecular weight within the range of from 100,000 to 10,000,000 Dalton, preferably within the range of from 500,000 to 5,000,000 Dalton, more preferably within the range of from 800,000 to 4,000,000 Dalton, and most preferably within the range of from 1,000,000 to 3,000,000 Dalton. In another example, the ophthalmic composition may contain from 0.02 to 0.04 % by weight of one or more viscosity regulating agent, preferably of hyaluronic acid and/or a hyaluronate, having a molecular weight within the range of from 100,000 to 10,000,000 Dalton, preferably within the range of from 500,000 to 5,000,000 Dalton, more preferably within the range of from 800,000 to 4,000,000 Dalton, and most preferably within the range of from 1 000,000 to 3,000,000 Dalton.

In one embodiment, the weight average molecular weight of the at least one viscosity regulating agent (preferably of the hyaluronic acid or hyaluronate) is within the range of from 100,000 to 10,000,000 Dalton (100 kDa to 10 MDa), preferable within the range of from 500,000 to 5,000,000 Dalton (500 kDa to 5 MDa), more preferably within the range of from 800,000 to 4,000,000 Dalton (800 kDa to 4 MDa), and most preferably within the range of from 1,000,000 to 3,000,000 Dalton (1 MDa to 3 MDa). The weight average molecular weight may be determined by size exclusion chromatography (SEC) in combination with multi angle laser light scattering (MALLS) detector (SEC-MALS).

In a preferred embodiment, the ophthalmic composition of the invention comprises hyaluronic acid or a salt thereof as the at least one viscosity regulating agent, and the weight average molecular weight of the hyaluronic acid or salt thereof is within the range of from 100,000 to 10,000,000 Dalton, preferable within the range of from 500,000 to 5,000,000 Dalton, more preferably within the range of from 800,000 to 4,000,000 Dalton, even more preferably within the range of from 1,000,000 to 3,000,000 Dalton, further more preferably within the range of from 1,300,000 to 2,600,000 Dalton, and most preferably within the range of from 1,600,000 to 2,400,000 Dalton. The weight average molecular weight may be determined by SEC-MALS.

GPC-MALLS is widely used to characterize high molecular weight (MW) polymers, which uses a differential refractometer and a multi-angle laser light scattering detector (MALLS or MALS) as detectors for size exclusion chromatography (also referred to as gel permeation chromatogram (GPC) in the art). Separation of polymers is achieved by SEC based on different MW. The average molecular weight of a polymer is determined by MALLS based the differential scattering extent/angle of molecules of different MW. The SEC-MALLS method allows continuous measurement of the molecular weight and radius of gyration of each fraction separated by SEC. Principles of SEC-MALLS and protocols suited for hyaluronic acid and other viscosity regulating agents are described by Ueno et al., 1988, Chem. Pharm. Bull. 36, 4971-4975; Wyatt, 1993, Anal. Chim. Acta 272: 1-40; and Wyatt Technologies, 1999, "Light Scattering University DAWN Course Manual" and "DAWN EOS Manual" Wyatt Technology Corporation, Santa Barbara, Calif.

The dynamic viscosity of the ophthalmic composition of the invention may be from 1 to 10, preferably from 2 to 5 cP (mPa s) at 20°C determined using a rotational concentric cylinder viscometer at 100 s⁻¹ according to Ph. Eur. 2.2.10.

The ophthalmic emulsion of the invention may further contain suitable additives or pharmaceutical excipients. Examples of additives or excipients are stabilisers such as EDTA; or preservatives. Examples of preservatives frequently used in ophthalmic compositions are quaternary ammonium compounds such as benzalkonium chloride, benzethonium chloride, or polyquat. However, preferably, the ophthalmic composition of the invention does not contain a preservative, more preferably it does not contain a quaternary ammonium compound as preservative. Any preservative present may be contained at less than 0.001 % by weight of a preservative, preferably of a quaternary ammonium compound preservative, such as benzalkonium chloride.

The ophthalmic emulsion of the invention may further contain an active pharmaceutical ingredient, such as an antibiotic, a vitamin, such as Vitamin E, an antiphlogistic agent, an analgesic agent, and/or an agent for decreasing intra-ocular pressure or for treatment of glaucoma. Examples of an antibiotic are chloramphenicol, ofloxacin, norfloxacin, moxifloxacin, and neomycin. Examples of vitamins are retinol, retinol acetate, retinol palmitate, tocopherol acetate, or D-α-tocopherol polyethylene glycol (TPGS). An example of an analgesic agent is flurbiprofen. Examples of an agent for decreasing intra-ocular pressure or for treatment of glaucoma are bimatoprost, latanoprost, travoprost, brinzolamide, and timolol, or combinations thereof. In case such active agents are present in the emulsion of the invention, the emulsion may be used in the treatment of the respective disease, such as ocular infection, increased ocular pressure, or glaucoma.

The ophthalmic emulsion is for use in ophthalmology. The ophthalmic composition may be for use in a method of treating, preventing or reducing dry eye symptoms and/or dry eye disease. Such symptoms include foreign body sensation/feels like something is in the eyes, eyes feel 'gritty' - often worse in the mornings, blurred vision, burning sensation in eyes, irritable eyelids, light sensitivity, redness of the whites of the eyes, painful eyes, excessive watering, and diseases include keratoconjunctivitis sicca, meibomian gland dysfunction, Sjogren's syndrome, postoperatively, e.g. after cataract surgery, LASIK, LASEK, caused by environmental stress, tiredness and visual stress due to intense vision, e.g. on computer screens, microscopes or long car journeys, caused by mechanical stress, e.g. by wearing hard or soft contact lenses or through diagnostic interventions on the eye. The dry eye symptoms may be light, moderate or severe. Use of the composition according to the invention may lead to improvements in eye symptoms, for example to improvement in subjective comfort (more), eye redness (less), eye irritation or burning (less), tear film deficiencies (corrected) or tear break-up time (TBUT, minimally 15 seconds). The composition may be applied to the closed eye lid (spray) or to the inner side of the lower eye lid (drops). The drops may then be introduced into the eye by movement of the eye lid. The composition may advantageously be used by contact lens wearers, people with allergy and people over 40, which groups more frequently than other people experience dry eye problems or problems of burning or itching eyes.

The ophthalmic emulsion of the present invention is generally applied topically to a subject's eye affected by any of the indications mentioned above. The ophthalmic composition may be applied to an eye, such as the conjunctival sac of an eye, of a subject in need thereof. The composition may be applied in a single dose. However, preferably, it is applied two or more times per day, preferably three to five times per day. It may be applied to an eye one or more times per day over a period of from one day to several weeks (e.g. to four weeks), such as from one day to two weeks or from two days to seven days. The ophthalmic emulsion may be applied for periods even longer than four weeks. If the ophthalmic emulsion is formulated as an eye drop formulation, it may be applied by dropwise instillation into an eye, such as into the conjunctival sac of an eye.

The process of the invention for the preparation of an ophthalmic oil-in-water emulsion comprises the steps of mixing at least components a) to d), namely a) at least one ophthalmically acceptable oil, wherein the ophthalmically acceptable oil comprises castor oil, b) tyloxapol, c) poloxamer 188, and d) polyoxyethylene (20) sorbitan mono-oleate; and emulsifying the mixture by high shear mixing to obtain an emulsion, preferably a nano emulsion. As to the amounts of the compounds a) to d) as defined above, these may be used as in the emulsion as preferably described above, i.e. for the final ophthalmological oil-in-water emulsion. Preferably, in the process, a mixture is emulsified, which is a concentrate, preferably a 8 to 20-fold, more preferably a 10 to 16 fold, concentrate of the emulsion as defined above.

That is, in the concentrate, preferably the content of the tyloxapol is about 0.8 wt% to about 20 wt%, more preferably about 1.6 wt% to about 12 wt %, in particular about 2 wt% to about 10 wt%, e.g. about 3 wt% to about 8 wt%.

In the concentrate, preferably the content of poloxamer 188 is about 0.24 wt% to about 20 wt%, more preferably about 0.4 wt% to about 10 wt %, in particular about 0.64 wt% to about 4 wt%, e.g. about 1 wt% to about 2 wt%.

In the concentrate, preferably the content of polyoxyethylene (20) sorbitan monooleate is about 0.40 wt% to about 20 wt%, more preferably about 0.8 wt% to about 12 wt %, in particular about 1.2 wt% to about 6 wt%, e.g. about 2 wt% to about 4 wt%.

In the concentrate, preferably the content of the cosurfactant, preferably of glycerol, is about 0.8 wt% to about 60 wt%, more preferably about 4 wt% to about 50 wt %, in particular about 8 wt% to about 40 wt%.

Preferably, no high pressure homogenization step, i.e. a homogenization step using pressures of more than 500 bar, e.g. by using Avestin Emulsiflex C3 Homogenizer or equivalent equipment is used in the process. A typical high pressure homogenization step usually involves several passages using pressures of 500 to 1500 bar or higher.

Typically, in the process of the invention, compounds a) to d), preferably as first step, the surfactants b), c) and d) and more preferably also a cosurfactant, most preferably glycerol, are mixed, followed by addition of the oil a). Preferable, the step of addition of oil is conducted under inert atmosphere, preferably nitrogen atmosphere, and/or reduced pressure, e.g. at or below 0.1 bar.

Preferably, the aqueous phase, most preferably water, which may include salts and buffers, is added during the emulsifying step. The emulsion is typically prepared using a homogenizer, such as a rotor-stator homogenizer. An important performance parameter in rotor-stator homogenizers is the tip speed of the rotor (peripheral velocity). This parameter is a function both of rotation speed and of rotor diameter. A tip speed of at least 10 ms⁻¹ is useful, and ideally faster e.g. ≥20 ms⁻¹ , ≥50 ms⁻¹ , ≥100 ms⁻¹ , etc. A tip speed of 50 ms⁻¹ or higher can be readily achieved at 10,000 rpm with a small homogenizer or at lower rotation speeds (e.g. 1,000 to 2,000 rpm) with a larger homogenizer. Suitable high-shear homogenizers are commercially available. It has been found that such homogenizers are sufficient to provide a suitable nano emulsion of the invention, and that more sophisticated devices, such as high pressure homogenizers using pressures of at least 500 bar (50 MPa), are not necessary with the specific surfactant system used.

In general, the process of the invention is performed between 20°C to 60°C, more preferably at 40°C to 60°C. Typically, the operation time or the homogenizer required to obtain a suitable emulsion is 30 to 120 minutes.

The present invention further relates to an ophthalmic oil-in-water emulsion concentrate as defined above, in particular obtainable by a process as defined above. The concentrate, upon dilution, e.g. by a factor of 8 to 20, allows providing an ophthalmic oil-in-water emulsion is as defined above. Thus, the present invention relates to an ophthalmic oil-in-water emulsion comprising the components in the amounts as defined above, and additionally a concentrate, preferably a 8 to 20 fold, more preferably a 10 to 16 fold concentrate. Such concentrate thus comprises the same kinds of the components of the emulsion of the invention, but in an 8 to 20 fold, more preferably a 10 to 16 fold concentration.

Such concentrate provides, upon dilution with water, the ophthalmic oil-in-water emulsion comprising the components in the amounts as defined above. In order to further reduce the device-intensity of the production process of emulsions, as the emulsion of the invention, it has been found to be advantageous to prepare a concentrate of the emulsion, and after emulsification (homogenisation), which provides the desired nano emulsion, to dilute the concentrate to obtain the final product. In particular, it has been surprisingly found that when the surfactant system of the invention is used, the oil droplet size is substantially kept constant upon dilution, i.e. the nano emulsion substantially stays a nano emulsion, compared to when a surfactant system as known in the art, e.g. only comprising tyloxapol and a poloxamer, is used. This is also illustrated in the Examples below.

### EXAMPLES

The present invention will next be described in more detail by way of Examples, which should not be construed as limiting the invention thereto.

### HLB value measurement

The HLB number of an unknown surfactant is generally determined by experimental measurement adapted from the method developed by Griffin (J Soc Cosmetic Chemists 1, 311 326 (1949)), i.e. the "conventional method" as described in Nollet et al, International Journal of Cosmetic Science, 2019, 41, 99-10.

In the experimental measurement approach, the tested surfactant (referred to as "S") is coupled in different proportions with a reference surfactant. Span 80 (HLB = 4.3) (Sorbitan-monooleat, CAS 1338-43-8) and Tween 80 (HLB = 15) are chosen as reference surfactants. These are well-documented and have been used in cosmetic and pharmaceutical formulations for many years. If the expected HLB number of 'S' is high, it is combined with Span 80. If HLB number is low it is combined with Tween 80. Emulsions are prepared with these mixtures, water and paraffin oil (required HLB about 10). The composition of the emulsions prepared comprises 5% of surfactant (or a surfactant mixture), 15% of oil and 80% of water. The hydrophilic surfactant is solubilized in water and the lipophilic one in oil. Each phase is heated to 80°C for 5 min. The oil phase is dispersed in the aqueous phase at 900 rpm (using a Turbotest homogenizer from VMI Rayneri) for 15 min; then stirred at 600 rpm for six minutes in a 25°C water bath. At this point, a watersoluble dye (FD&C Blue No. 1) is added to enable the visual comparison of the stability of each emulsion. Emulsion stability is evaluated by observing the creaming of the emulsion. The more stable the emulsion the less macroscopic phase separation and vice versa. The more stable emulsion contains the surfactant mixture with an HLB number equal to 10. The HLB number of the tested surfactant is then calculated using the equation: 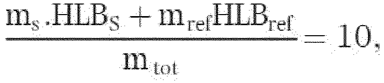 where m_{ref} and HLB_{ref} are the mass and the HLB of Span 80 or Tween 80, respectively. mₛ is the mass of the tested surfactant and mₜₒₜ = mₛ + m_{ref} .

### Measurement of pH

The pH is determined by potentiometric determination according to EP2.2.3 on a 10 ml sample at 20°C.

### Measurement of oil droplet size

The oil droplet size is determined by a dynamic light scattering by a submicron particle size analyzer N5 Beckman Coulter equipped with PCS control Software

### Run Parameters:

- Equilibration Time 2 minutes.
- Repetition numbers: 1
- Angle 90°
- Runtime: manual, 300 s
With respect to particle size distribution characterization, a parameter used to define the size range of the lipidic nanocarrier systems is called the "polydispersity index" (PDI). The term "polydispersity" (or "dispersity" as recommended by IUPAC) is used to describe the degree of non-uniformity of a size distribution of particles. Also known as the heterogeneity index, PDI is a number calculated from a two-parameter fit to the correlation data (the cumulants analysis). This index is dimensionless and scaled such that values smaller than 0.05 are mainly seen with highly monodisperse standards. PDI values bigger than 0.7 indicate that the sample has a very broad particle size distribution and is probably not suitable to be analysed by the dynamic light scattering (DLS) technique. Different size distribution algorithms work with data that fall between these two extreme values of PDI i.e., 0.05-0.7). The calculations used for the determination of size and PDI parameters are defined in the ISO standard documents 13321:1996 E and ISO 22412:2008.

### Measurement of dynamic viscosity

The determination of the dynamic viscosity for the ophthalmic solutions is carried out by means of a rotational concentric cylinder viscometer (Searle type) at 20°C according to EP 2.2.10.

### Materials

- Metallic cylinder supplied with the viscosimeter
- Rotational viscosimeter Brookfield DV3T-Rheometer or equivalent
- Spindle: SC4-18

### Method

- Temperature 20°C.
- Pour into the 16ml metallic cylinder the test solution, sample size 8 ml.
- Insert the cylinder in the dedicated section on the base of the viscosimeter.
- Connect the temperature sensor to the viscosimeter.
- Place the viscosimeter onto the base.
- Turn on the instrument
- Speed 250 rpm.

The measurement is performed three times with the same sample. Reported values are the average of the three measurements.

### Measurement of osmolality

Osmolality is determined by measuring freezing point depression according to Eur. Pharm. 2.2.35. A micro-osmometer Löser Type6/6M was used. The method is carried out as described by the manufacturer as follows:
1. Turn on the instrument and wait for the acoustic signal.
2. Fill the centrifuge tube (1.5 ml) with 100 µl of test solution and place it in the thermistor (temperature dependent resistor).
3. Pull down the thermistor in the cooling hole.
4. The value of the decreasing temperature is displayed. It ensures the progressive freezing. After 1.5 minutes the acoustic signal indicates that the freezing point is reached.
5. Raise completely the freezing needle and insert it into the tube. After 1 second replace the needle to original position.
6. Wait for the acoustic signal and record the value shown on the display.
7. Remove the sample tube.

The measurement for the analytical data determination is performed three times with the same sample/ flask, and we report the average.

The measurement is performed three times with the same sample. Reported values are the average of the three measurements.

### EXAMPLE 1

A concentrate emulsion of the invention as well as a comparative emulsion 1 (without the third surfactant, here Tween 80; the amounts used for the remaining ingredients were adjusted to compensate for the removal of this component) were prepared having the following composition:

### Composition:

| | **Emulsion** | **Comparative Emulsion 1** |
|---|---|---|
| **Component** | **[wt%]** | **[wt%]** |
| Castor oil | 15 | 15.56 |
| Tyloxapol | 5 | 5.2 |
| Poloxamer 188 | 1.67 | 1.72 |
| Tween 80 | 3.33 | --- |
| Glycerol | 26.67 | 27.56 |
| Water | ad 100 % | ad 100 % |

The emulsions were prepared as follows:
Glycerol (cosurfactant, glycerol anhydrous) and the surfactants (tyloxapol, poloxamer 188, and if present, Tween 80; all pharmagrade) were weighted in a working basin and the temperature of the water bath was set to 60°C. The components were mixed for 10 minutes.

The ophthalmically acceptable oil (castor oil) was poured into the basin after stopping the mixer, followed by a nitrogen wash. The oil is mixed with the other components under reduced pressure (below 0.1 bar) for 10 minutes.

Water having a temperature of 60°C was added, and at the moment when the water reaches the mixture of oil and surfactants, the mixer is set to at least 1000 rpm. The amount of water is added in about 10 minutes, while the temperature is kept at 60°C (cooler bath set to 42°C +/-2°C). These conditions are maintained for 2 hours.

For the following cooling step, the mixer is set to about 200 rpm, and the cooler temperature is set to 20°C, until the product reaches 36°C (about 1 hour). The product is then withdrawn under nitrogen atmosphere.

### EXAMPLE 2

To prepare the final emulsion, the concentrate is diluted about 16 fold using Tris buffer (about 0.08 wt%) having a pH of 7.1.

The following properties of the emulsions were determined:

| | **Emulsion** | **Comparative Emulsion 1** |
|---|---|---|
| **Before dilution** | | |
| Oil droplet size | 196 nm | 281 nm |
| PDI | 0.296 | 0.621 |

| **After dilution** | | |
|---|---|---|
| Oil droplet size | 176 nm | 757 nm |
| PDI | 0.315 | 0.693 |
| pH | 7.13 | 7.15 |

For comparison, the concentrate comparative emulsion 1 was also diluted with Tris buffer containing 0.2 wt% Tween 80. However the oil droplet size and the PDI were similar to the comparative emulsion after dilution.

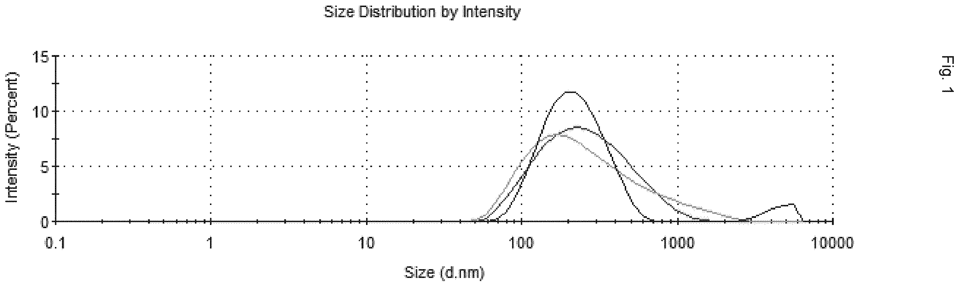

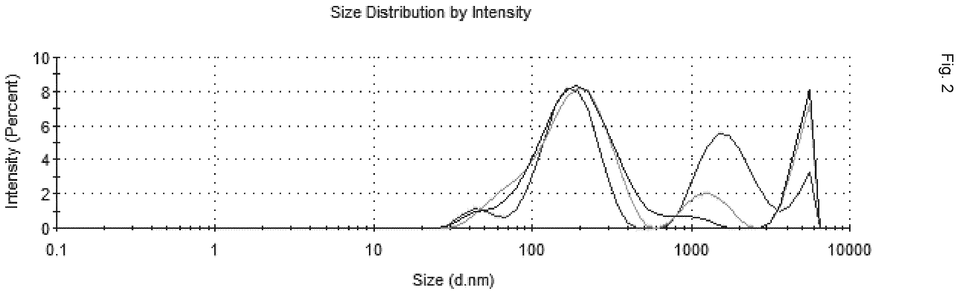

Thus, the surfactant system of the invention allows preparing an oil-in-water concentrate as well as an emulsion having excellent oil droplet size (nano emulsion) and a narrow droplet size distribution (cf. Fig. 1) without the necessity of using expensive high pressure homogenization. The comparative emulsion shows significantly inferior properties (cf. Fig. 2).

### EXAMPLE 3

A concentrate emulsion of the invention as well as a comparative emulsion 2 (comprising a third surfactant having an HLB of 8.6, i.e. Span 20 (Sorbitan monolaurate)) were prepared on a small scale using a lab emulsifier (10000 rpm).

The following properties of the emulsions were determined:

| | **Emulsion** | **Comparative Emulsion 2** |
|---|---|---|
| Oil droplet size | 160 nm | 241 nm |
| PDI | 0.211 | 0.349 |

Thus, the surfactant system of the invention allows preparing an oil-in-water concentrate as well emulsion having excellent oil droplet size (nano emulsion) and a narrow droplet size distribution, which is superior compared to using a different third surfactant.

### EXAMPLE 4

The stability of an emulsion of the invention was tested in a 6 months (180 days) test. 10 ml Samples were stored in polyethylene bottles at 25°C ±2°C/40%±5% RH and 40°C±2°C/25%RH, respectively.

The results are summarized in the following Table:

| | T=0 | 6M (25±2°C/40±5% RH) | 6M (40±2°C/25% RH) |
|---|---|---|---|
| pH | 7.52 | 7.47 | 7.51 |
| Osmolality | 245 mOsm/kg | 246 mOsm/kg | 247 mOsm/kg |
| Droplet size | 167 nm | 156 nm | 172 nm |

As confirmed by the stability test, the emulsion of the invention shows excellent stability upon 6 months storage even under demanding conditions.

## Claims

1. An ophthalmic oil-in-water emulsion comprising:
a) at least one ophthalmically acceptable oil, wherein the ophthalmically acceptable oil comprises castor oil,
b) tyloxapol,
c) poloxamer 188, and
d) polyoxyethylene (20) sorbitan mono-oleate.

2. The ophthalmic oil-in-water emulsion according to claim 1, wherein the content of the ophthalmically acceptable oil is about 0.5 to 2 wt%, the content of tyloxapol is about 0.1 to about 1 wt%, and the content of the poloxamer having an HLB value equal to or higher than 18 is about 0.05 to about 1 wt%.

3. The ophthalmic oil-in-water emulsion according to claim 1 or 2, wherein the ophthalmically acceptable oil is substantially free of mineral oil and medium chain triglycerides.

4. The ophthalmic oil-in-water emulsion according to any of the preceding claims, wherein the ophthalmically acceptable oil substantially consists of castor oil.

5. The ophthalmic oil-in-water emulsion according to any of the preceding claims, wherein the non-ionic surfactant of d) is present in an amount of about 0.05 to 0.5 wt%.

6. The ophthalmic oil-in-water emulsion according to any of the preceding claims, wherein the emulsion further comprises at least one cosurfactant, preferably glycerol.

7. The ophthalmic oil-in-water emulsion according to any of the preceding claims, wherein the emulsion further comprises at least one osmolarity agent.

8. The ophthalmic oil-in-water emulsion according to any of the preceding claims, wherein the emulsion further comprises at least one or more viscosity regulating agent selected from the group consisting of a hydroxyalkylated cellulose, chondroitin sulfate, polyacrylic acid, polyvinyl alcohol, polyethylene glycol, polysaccharides, polyvinylpyrrolidone, hyaluronic acid, cross-linked hyaluronic acid, and salts thereof, preferably hyaluronic acid and salts thereof, preferably in an amount of from 0.001 to 0.1 wt%, preferably from 0.015 to 0.06 wt%, more preferably from 0.02 to 0.04 wt%, and most preferably from 0.025 to 0.035 wt%.

9. The ophthalmic oil-in-water emulsion according to any of the preceding claims, wherein the emulsion further comprises at least one or more osmoprotectants selected from the group consisting of glycine betaine, myo-inositol, taurine, ectoine and/or trimethylamine-N-oxide.

10. A process for the preparation of an ophthalmic oil-in-water emulsion comprising the steps of
mixing at least components a) to d):
a) at least one ophthalmically acceptable oil, wherein the ophthalmically acceptable oil comprises castor oil,
b) tyloxapol ,
c) poloxamer 188, and
d) polyoxyethylene (20) sorbitan mono-oleate; and
emulsifying the mixture using high shear mixing to obtain a nano emulsion.

11. The process according to claim 10, wherein the ophthalmic oil-in-water emulsion is as defined in any one of claims 1 to 9.

12. An ophthalmic oil-in-water emulsion concentrate obtainable by a process according to claim 10, which upon dilution, provides an ophthalmic oil-in-water emulsion as defined in any one of claims 2 to 9.

## Patentansprüche

1. Eine ophthalmische Öl-in-Wasser-Emulsion, die Folgendes umfasst:
a) mindestens ein ophthalmisch akzeptables Öl, wobei das ophthalmisch akzeptable Öl Rizinusöl umfasst,
b) Tyloxapol,
c) Poloxamer 188, und
d) Polyoxyethylen(20)sorbitanmonooleat.

2. Die ophthalmische Öl-in-Wasser-Emulsion nach Anspruch 1, wobei der Gehalt an ophthalmisch verträglichem Öl etwa 0,5 bis 2 Gew.-%, der Gehalt an Tyloxapol etwa 0,1 bis etwa 1 Gew.-% und der Gehalt an Poloxamer mit einem HLB-Wert von 18 oder mehr etwa 0,05 bis etwa 1 Gew.-% beträgt.

3. Die ophthalmische Öl-in-Wasser-Emulsion nach Anspruch 1 oder 2, wobei das ophthalmisch akzeptable Öl im Wesentlichen frei von Mineralöl und mittelkettigen Triglyceriden ist.

4. Die ophthalmische Öl-in-Wasser-Emulsion nach einem der vorhergehenden Ansprüche, wobei das ophthalmisch akzeptable Öl im Wesentlichen aus Rizinusöl besteht.

5. Die ophthalmische Öl-in-Wasser-Emulsion nach einem der vorhergehenden Ansprüche, wobei das nichtionische Tensid d) in einer Menge von etwa 0,05 bis 0,5 Gew.-% vorhanden ist.

6. Die ophthalmische Öl-in-Wasser-Emulsion nach einem der vorhergehenden Ansprüche, wobei die Emulsion außerdem mindestens ein Co-Tensid, vorzugsweise Glycerin, enthält.

7. Die ophthalmische Öl-in-Wasser-Emulsion nach einem der vorhergehenden Ansprüche, wobei die Emulsion außerdem mindestens ein Osmolaritätsmittel enthält.

8. Die ophthalmische Öl-in-Wasser-Emulsion nach einem der vorhergehenden Ansprüche, wobei die Emulsion ferner mindestens ein oder mehrere viskositätsregulierende Mittel enthält, die aus der Gruppe ausgewählt sind, die aus hydroxyalkylierter Cellulose, Chondroitinsulfat, Polyacrylsäure, Polyvinylalkohol, Polyethylenglykol, Polysacchariden, Polyvinylpyrrolidon, Hyaluronsäure, vernetzter Hyaluronsäure und Salzen davon besteht, vorzugsweise Hyaluronsäure und Salzen davon, vorzugsweise in einer Menge von 0,001 bis 0,1 Gew.-%, vorzugsweise von 0,015 bis 0,06 Gew.-%, noch bevorzugter von 0,02 bis 0,04 Gew.-% und am bevorzugtesten von 0,025 bis 0,035 Gew.-%.

9. Die ophthalmische Öl-in-Wasser-Emulsion nach einem der vorhergehenden Ansprüche, wobei die Emulsion außerdem mindestens ein oder mehrere Osmoprotektiva umfasst, die aus der Gruppe ausgewählt sind, die aus Glycinbetain, Myo-Inositol, Taurin, Ectoin und/oder Trimethylamin-N-oxid besteht.

10. Verfahren zur Herstellung einer ophthalmischen Öl-in-Wasser-Emulsion, das die folgenden Schritte umfasst
Mischen mindestens der Komponenten a) bis d):
a) mindestens ein ophthalmisch akzeptables Öl, wobei das ophthalmisch akzeptable Öl Rizinusöl umfasst,
b) Tyloxapol,
c) Poloxamer 188, und
d) Polyoxyethylen(20)-sorbitanmonooleat; und
Emulgieren der Mischung durch Mischen unter hoher Scherung, um eine Nanoemulsion zu erhalten.

11. Das Verfahren nach Anspruch 10, wobei die ophthalmische Öl-in-Wasser-Emulsion wie in einem der Ansprüche 1 bis 9 definiert ist.

12. Ophthalmisches Öl-in-Wasser-Emulsionskonzentrat, erhältlich durch ein Verfahren nach Anspruch 10, das nach Verdünnung eine ophthalmische Öl-in-Wasser-Emulsion, wie in einem der Ansprüche 2 bis 9 definiert, liefert.

## Revendications

1. Émulsion ophtalmique huile-dans-eau comprenant :
a) au moins une huile ophtalmiquement acceptable, dans laquelle l'huile ophtalmiquement acceptable comprend l'huile de ricin,
b) du tyloxapol,
c) du poloxamer 188, et
d) du monooléate de polyoxyéthylène (20) sorbitane.

2. Émulsion ophtalmique huile-dans-eau selon la revendication 1, dans laquelle la teneur en huile ophtalmiquement acceptable est d'environ 0,5 à 2 % en poids, la teneur en tyloxapol est d'environ 0,1 à environ 1 % en poids, et la teneur en poloxamer ayant une valeur HLB supérieure ou égale à 18 est d'environ 0,05 à environ 1 % en poids.

3. Émulsion ophtalmique huile-dans-eau selon la revendication 1 ou 2, dans laquelle l'huile ophtalmiquement acceptable est sensiblement exempte d'huile minérale et de triglycérides à chaîne moyenne.

4. Émulsion ophtalmique huile-dans-eau selon l'une des revendications précédentes, dans laquelle l'huile ophtalmiquement acceptable est sensiblement composée d'huile de ricin.

5. Émulsion ophtalmique huile-dans-eau selon l'une des revendications précédentes, dans laquelle le tensioactif non ionique de d) est présent en une quantité d'environ 0,05 à 0,5 % en poids.

6. Émulsion ophtalmique huile-dans-eau selon l'une des revendications précédentes, dans laquelle l'émulsion comprend en outre au moins un co-tensioactif, de préférence un glycérol.

7. Émulsion ophtalmique huile-dans-eau selon l'une des revendications précédentes, dans laquelle l'émulsion comprend en outre au moins un agent d'osmolarité.

8. Émulsion ophtalmique huile-dans-eau selon l'une des revendications précédentes, dans laquelle l'émulsion comprend en outre au moins un ou plusieurs agents de régulation de la viscosité sélectionnés parmi le groupe consistant en une cellulose hydroxyalkylée, une chondroïtine sulfate, un acide polyacrylique, un alcool polyvinylique, un polyéthylène glycol, des polysaccharides, une polyvinylpyrrolidone, un acide hyaluronique, un acide hyaluronique réticulé et les sels de ceux-ci, de préférence un acide hyaluronique et les sels de celui-ci, de préférence en une quantité de 0,001 à 0,1 % en poids, de préférence de 0,015 à 0,06 % en poids, de manière davantage préférée de 0,02 à 0,04 % en poids, et de manière préférée entre toutes de 0,025 à 0,035 % en poids.

9. Émulsion ophtalmique huile-dans-eau selon l'une des revendications précédentes, dans laquelle l'émulsion comprend en outre au moins un ou plusieurs osmoprotecteurs sélectionnés parmi le groupe consistant en une glycine bétaïne, un myoinositol, une taurine, une ectoïne et/ou un triméthylamine-N-oxyde.

10. Procédé de préparation d'une émulsion ophtalmique huile-dans-eau comprenant les étapes consistant à mélanger au moins les composants a) à d) :
a) au moins une huile ophtalmiquement acceptable, dans laquelle l'huile ophtalmiquement acceptable comprend l'huile de ricin,
b) du tyloxapol,
c) du poloxamer 188, et
d) du monooléate de polyoxyéthylène (20) sorbitane ;
et à émulsifier le mélange en utilisant un mélange à cisaillement élevé pour obtenir une nanoémulsion.

11. Procédé selon la revendication 10, dans lequel l'émulsion ophtalmique huile-dans-eau est telle que définie dans l'une des revendications 1 à 9.

12. Concentré d'émulsion ophtalmique huile-dans-eau pouvant être obtenu par un procédé selon la revendication 10, qui après dilution, fournit une émulsion ophtalmique huile-dans-eau telle que définie dans l'une des revendications 2 à 9.
